# EUROPEAN PATENT SPECIFICATION

(11) **EP 3 937 903 B1**
(45) Date of publication and mention of the grant of the patent: **28.10.2026**
(21) Application number: 20713380.2
(22) Date of filing: 10.03.2020
(51) Int. Cl.: A61K 31/5383, A61K 9/00, A61P 31/04, A61P 29/00, A61P 27/02, A61K 31/573

(54) **REDUCTION OF ANTIBIOTIC DOSAGE IN ANTIBIOTIC/ANTI-INFLAMMATORY COMPOSITIONS COMBINED TOGETHER FOR OPHTHALMIC USE**
VERRINGERUNG DER ANTIBIOTISCHEN DOSIERUNG BEI ANTIBIOTISCHEN/ANTIINFLAMMATORISCHEN ZUSAMMENSETZUNGEN IN KOMBINATION ZUR ANWENDUNG AM AUGE
RÉDUCTION DE DOSAGE D'ANTIBIOTIQUE DANS DES COMPOSITIONS ANTIBIOTIQUES/ANTI-INFLAMMATOIRES COMBINÉES POUR UNE UTILISATION OPHTALMIQUE

(30) Priority: 11.03.2019 IT 201900003529
(43) Date of publication of application: 19.01.2022
(73) Proprietor: NTC S.r.l., 20124 Milano (IT)
(72) Inventor: CARNOVALI, Marino, 20124 Milano (IT); MARCELLONI, Luciano, 20124 Milano (IT); BERTOCCHI, Federico, 20124 Milano (IT)
(74) Representative: Benedetto, Marco
(86) International application number: PCT/IB2020/052057
(87) International publication number: WO 2020/183361

(56) References cited:
- EP-A1- 3 216 451
- WO-A1-2014/190248
- WO-A1-89/09057
- DE-A1- 19 729 879
- O YILDIRIM ET AL: "The efficacy of intravitreal levofloxacin and intravitreal dexamethasone in experimental Staphylococcus epidermidis endophthalmitis", 1 January 2002 (2002-01-01), XP055471835, Retrieved from the Internet <URL:http://www.ncbi.nlm.nih.gov/pubmed/12483022> [retrieved on 20180502]
- ANONYMOUS: "History of Changes for Study: NCT03739528", 30 November 2018 (2018-11-30), XP055640103, Retrieved from the Internet <URL:https://clinicaltrials.gov/ct2/history/NCT03739528?V_2=View#StudyPageTop> [retrieved on 20191107]
- ESCRS ENDOPHTHALMITIS STUDY GROUP: "Prophylaxis of postoperative endophthalmitis following cataract surgery: Results of the ESCRS multicenter study and identification of risk factors", JOURNAL CATARACT AND REFRACTIVE SURGERY, SURGERY, FAIRFAX, VA, US, vol. 33, no. 6, 23 May 2007 (2007-05-23), pages 978 - 988, XP022093374, ISSN: 0886-3350, DOI: 10.1016/J.JCRS.2007.02.032

## Description

The present invention regards an ophthalmic composition comprising a mixture comprising, a or alternatively, consisting of an antibiotic and a corticosteroid, and optionally one or more pharmaceutical grade additives and excipients; said composition being for use in a method for the prophylaxis of post-surgical infections and the treatment of inflammatory events in a patient previously subjected to eye surgery, preferably cataract surgery. The present invention regards a method for reducing antibiotic dosage in antibiotic/anti-inflammatory compositions combined together for ophthalmic use. The present invention relates to a method of administering said composition for use.

### FIELD OF THE INVENTION

Eye disorders often have a combination of inflammatory and infectious aspects that are concomitant with each other.

Going from the innermost area of the eye to the outermost one we find the following well delineated physical regions: retina, vitreous body, cornea, crystalline lens, extraocular muscles, conjunctiva, lacrimal ducts. Some diseases regard the first areas of the eye among those indicated above which are mainly treated through surgery (apart from glaucoma, a condition caused by elevated intraocular pressure, which is also treated with suitable drugs).

However, eye surgery often requires a therapy for the prevention of infections in the pre- and post-operative phase, carried out with antibiotics and anti-inflammatories capable of also controlling pain using eye drops. Conjunctiva diseases of the lacrimal pathways are conveniently cured using drugs carried most of the time in eye drops and ointments, sometimes even using ocular inserts. The diseases most frequently treated with eye drops regarding which there is a specific indication are selected from among the group comprising: (i) Glaucoma, (ii) Conjunctivitis, (iii) Allergic conjunctivitis, (iv) infections of the external eye of bacterial origin, (v) Conjunctivitis of viral origin, (vi) Dry eye and (vii) Watery eye.

Forms of administration using eye drops are often the only forms of treatment and cure for certain diseases. However, the destiny of the drug carried through a drop administration of eye drops is unfortunately very variable. As a matter of fact, it happens that, due to the defence mechanisms of the eye - blinking and lachrymation - aimed at the constant expulsion of foreign bodies such as dust and fibres, most of the administered drop is expelled from the area of administration and lost. It is estimated that the part which is expelled and lost can be quantified on average at around 70% of the dispensed amount (in drops).

Sometimes it can be as much as 90% of the administered amount. Typically, the amount that can be administered by means of eye drops is comprised from 20 to 50 microliters per drop.

More typically, eye drops have a volume comprised from 30 to 35 microliters and they are administered in multiple administrations throughout the day. For each single administration, the most common dosage is one or two drops per eye, but there are some cases where multiple drops per single administration several times a day are required. Numerous administrations throughout the day, whose number can even reach 8-10/day, are often provided for in order to compensate the tear washout.

Another criticality concerns the place of dispensing (area of administration) of the drop, which can simply be the cornea of the eye or the conjunctival sac if a more abundant or extended stay is desired.

Bacterial infections may be superficial (conjunctivitis) or deep (keratitis) and require the use of antibiotics, anti-inflammatories and even pain killers at times.

As mentioned, eye surgery often requires a therapy for the prevention of infections in the pre- and post-operative phase, carried out with antibiotics and anti-inflammatories using eye drops.

In addition to treatments aimed at limiting and reducing infections, in the pre-operative phase, anti-infectives are also used in the treatments of infections following surgery (post-operative phase), one of the most common ones today being cataract surgery.

Usually prescribed in the post-operative treatment of cataract surgeries are 2 drugs to be taken simultaneously, one useful for reducing irritative events due to tissue cicatrisation and the other as an antibiotic, aimed at preventing bacterial infections.

In the context of the present invention, the term "prophylaxis" is used to indicate any medical procedure whose purpose is to avoid or prevent the spread of infections and diseases (preventive treatment).

### DESCRIPTION OF THE INVENTION

A drug called Tobradex^{®} that combines the anti-inflammatory steroid dexamethasone with the antibiotic tobramycin in single eye drops is already available on the market. Tobradex^{®} is recommended for the treatment of pre- and post-operative phases of cataract surgery.

A volume of 1 ml of Tobradex^{®} eye drops -suspension contains: tobramycin 3 mg, dexamethasone 1 mg. The other excipients are: benzalkonium chloride, edetate disodium, sodium chloride, sodium sulphate, tyloxapol, hydroxyethyl cellulose, purified water. The bottle is usually a 5 ml bottle. It is recommended to administer 1 or 2 drops 4-5 times a day. Fixed combinations, such as Tobradex^{®}, are forms of simultaneous administration of 2 active ingredients at a predetermined dosage which, with respect to the sum of the two single active ingredients, lead to a therapeutic benefit and also offer an undeniable comfort of use. However, a disadvantage in the administration of fixed combinations (associations/combinations of two active pharmaceutical ingredients (a) and (b)), lies in extending the administration of one of the two components (for example the active ingredient (a)) over time even if potentially no longer be useful or necessary, only so as to comply with the required proposed dosage of the other component (for example, the active ingredient (b)) so that the latter completes the dosage thereof and thus be effective.

The Tobradex^{®} eye drops based on tobramycin and dexamethasone are prescribed to be administered for 14 days in order to administer the antibiotic tobramycin for 14 days. All these antibiotic therapy days are not necessary. Furthermore, besides an overstimulation of tissues, such extended antibiotic therapy generates a potential antibiotic resistance with a high not strictly required environmental dispersion.

The prior art document EP3216451A1 describes an aqueous ophthalmic composition comprising levofloxacin (or a salt thereof, or a solvate thereof), dexamethasone (or an ester thereof, or a salt thereof), and one or more isotonic agents for the treatment of an inflammatory disease of the external ocular area, for example post-operative inflammation. This composition is not prescribed in relation to post-surgical infections, and the document explains that administration is not particularly limited over time, until desired efficacy is achieved.

The prior art document O YILDIRIM ET AL.: "The efficacy of intravitreal levofloxacin and intravitreal dexamethasone in experimental Staphylococcus epidermis endophthalmitis*"* illustrates (abstract) a study aimed at investigating whether an intravitreal injection of levofloxacin combined with dexamethasone shows greater efficacy than an intravitreal injection of levofloxacin alone in relation to post-operative complications such as a *Staphylococcus epidermidis* endophthalmitis. This document does not address the possible use of the aforementioned combination of levofloxacin with dexamethasone for administrations other than an intravitreal injection. Furthermore, the combination of levofloxacin and dexamethasone discussed therein is not suitable for use in a therapeutic method of treatment, since this document states that further experimental studies will be necessary to achieve an effective therapeutic treatment.

Anonymous document "History of changes for study: "NCT03739528", 30 November 2018 discloses an hypothetical study comparing eyedrops of combined levofloxacin + dexamethasone for 7 days followed by dexamethasone alone for an additional 7 days vs. tobramycin + dexamethasone for 14 days for the prevention and treatment of the inflammation and prevention and treatment of infection associated with cataract surgery in adults.

Document ESCRS ENDOPHTALMITIS STYDY GROUP: "Prophylaxis and post-operative endophthalmitis following cataract surgery: results of the ESCRS multi-center study and identification of risk factor" discloses the administration of 0.5% of levofloxacin eye-drops after cataract surgery for 7 days starting at the day of surgery.

Document DE19729879A1 discloses the use of diclofenac in combination with levofloxacin in the prophylaxis of post-surgical infections and inflammation of the eye.

Document WO8909057A1 discloses a composition comprising tobramycin and a steroid such as dexamethasone or fluorometholone for topical ophthalmic delivery.

Thus, there arises the problem related to resistance in bacterial strains (giving rise to antibiotic-resistant bacterial strains) which, in the long run, also causes antibiotics to become ineffective due to the inadequate and prolonged use as well as environmental dispersion. The World Health Organisation recommends reducing the amount of antibiotic administered in all therapies.

Therefore, the need is felt to be able to have a therapy for the prevention of infections in the pre-operative and post-operative phase which provides for a reduced antibiotic administration both in terms of the (absolute) amount administered to the patient subjected to eye surgery and in terms of days of administration of the antibiotic.

Furthermore, there is also felt the need to have a non-invasive treatment and method of treatment (therapy), useful and effective to be used, preferably in the pre-operative phases, to prevent bacterial infections, but also in the post-surgical phases following surgery to the eyes, for example cataract, to prevent and treat bacterial infections, which do not reveal the limits, drawbacks and problems still observed in the known treatments.

After a long and intense research and development activity, the Applicant developed an ophthalmic composition, preferably an ophthalmic solution, a treatment, a method of treatment and a method of administering said ophthalmic composition (therapy and dosage regimen) capable of providing an adequate response to the current limits, drawbacks and problems.

### The invention is defined by the appended claims.

Forming an object of the present invention is an ophthalmic composition having the characteristics as defined in the attached claims.

Preferred embodiments of the present invention will be outlined in the detailed description below by way of example and, therefore, not limiting the scope of protection of the present invention.
Figure 1 shows the structural formula of levofloxacin.
Figure 2 shows the structure form of dexamethasone disodium phosphate.
Figure 3 shows the study protocol used in the present invention.
Figure 4 shows the signs detected as the primary endpoint of the study: the observation of the absence of cells under *the slit lamp.* No cells under the *slit lamp.*
Figure 5 shows the signs detected as the primary endpoint of the study: observation of the absence of material under *the slit lamp* (AQUEOUS FLARE).
Figure 6 shows the signs detected as secondary endpoints: percentage of patients suffering from hyperaemia in the different stages of the study.
Figure 7 shows the symptoms detected as secondary endpoints: percentage of patients suffering from discomfort/irritation in the different stages of the study.

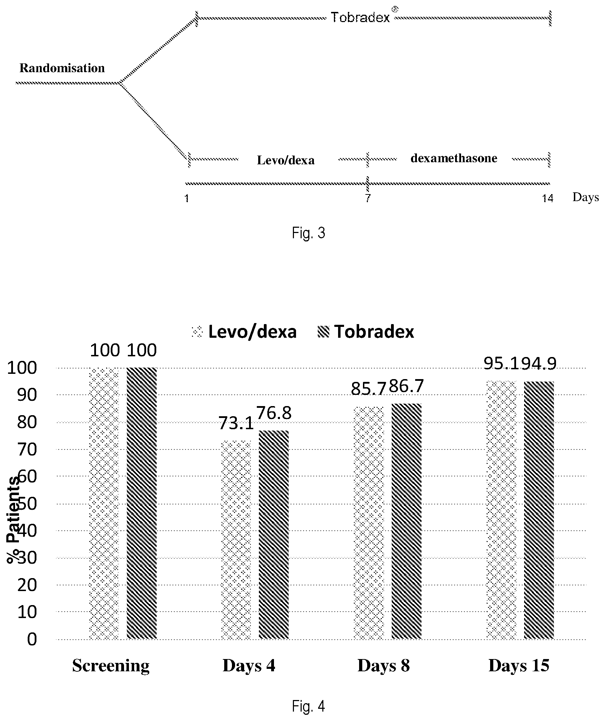

Below are the meanings of the expressions used in Figures 3 to 7: Levo/dexa is used to indicate the composition subject of the present invention such as for example the composition of Example 1.

The composition subject of the present invention is for use in a prophylaxis or in a prevention therapy or in a preventive treatment administered to a patient who has undergone (post-operative or post-surgical phase) eye surgery, for example to remove cataract.

The treatment and method of treatment of the present invention provide for the administration (method of administration) of a composition, preferably an ophthalmic composition. The composition of the present invention is a pharmaceutical composition. The composition of the present invention is a pharmaceutical form in eye drops in single-dose or multi-dose packaging, sterile solution or spray solution or ointment comprising a mixture comprising, a or alternatively, consisting of an antibiotic associated or combined with an anti-inflammatory.

The composition, the treatment and the method of treatment, which provide for the administration of said composition of the present invention, are effective in preventing, reducing or eliminating infections and/or inflammations to patients subjected to eye surgery, such as for example in the case of a patient subjected to cataract surgery, although being applied at a reduced dose regimen, with respect to similar treatments administered using Tobradex^{®} eye drops.

Advantageously, the treatment and the method of treatment which provide for the administration of the composition of the present invention (for example the compositions of Examples 1-4, see hereinafter), allow to reduce the administration of antibiotics to patients subjected to eye surgery, such as for example in case of intervention in a patient subjected to cataract surgery, by at least about 30%, or 40%, or 50%.

Advantageously, the treatment and the method of treatment of the present invention (which are carried out by administering the composition of the present invention) are capable of preventing the infective state within only 7 days of treatment; said treatment being followed, or preferably not followed, subsequently, only by a treatment of another 7 days based on the anti-inflammatory drug dexamethasone alone which allows to treat inflammatory events in a patient subjected to eye surgery.

Advantageously, the treatment and the method of treatment of the present invention (which are carried out by administering the composition of the present invention) are performed via non-intravitreal route.

In the present description, the expression "non-intravitreal" is used to indicate an administration in which neither an injection nor an implant of the composition subject of the present invention into the patient's eye are provided for. More precisely, such expression is used to indicate an administration in which neither the cornea, nor the lens, nor the sclera, nor the corneal limbus should be traversed through a mechanical action to place the composition object of the present invention into the vitreous body.

Advantageously, the treatment and the method of treatment of the present invention (which are carried out by administering the composition of the present invention) are performed via the ocular topical route. Preferably, the composition of the present invention is a pharmaceutical form in eye drops in single-dose or multi-dose packaging, sterile solution or spray solution or ointment comprising a mixture comprising, a or alternatively, consisting of an antibiotic associated or combined with an anti-inflammatory.

In an embodiment, the dosage and dose regimen are reported below. In the case of post-surgical prophylaxis, provided for is the administration of a composition of the present invention based on levofloxacin and dexamethasone to a patient for 7 days, for a number of times or daily administrations comprised from 1 to 4 times or administrations/day, advantageously 3 or 4 times a day, preferably 4 times or administrations/day, of 1 or 2 drops (preferably 1 drop), followed by 7 days of dexamethasone alone (without a subsequent administration of an antibiotic levofloxacin alone) or wherein, preferably, a subsequent administration of only one anti-inflammatory drug dexamethasone alone is not provided for and a subsequent administration of only one antibiotic levofloxacin antibiotic is not provided for. Therefore, in the latter embodiment, after 7 days administration of the composition of the present invention based on levofloxacin and dexamethasone to the patient, the dosage and dosage regimen do not envisage a further individual administration of levofloxacin or dexamethasone.

Forming an object of the present invention is (i) an ophthalmic composition in solution form comprising (ii) a mixture and, optionally, (iii) one or more pharmaceutical grade additives and/or excipients. The mixture (ii) comprises or, alternatively, consists of an (iia) antibiotic having a reduced bacterial resistance combined or associated with an anti-inflammatory (iib) selected from among corticosteroids. The composition (i) being for use in a method for the pre-surgical and post-surgical treatment of bacterial infections in a patient to be subjected to eye surgery. Eye surgery may be for removing cataract. The composition (i) is administered in the eye of said patient, after surgery. Being the composition (i) be administered after surgery, the treatment method provides for a daily mode for a period of time of 7 days subsequently to the eye surgery.

Furthermore, forming an object of the present invention is (i) an ophthalmic composition in solution form comprising (ii) a mixture and, optionally, (iii) one or more pharmaceutical grade additives and/or excipients and water. The mixture (ii) comprises or, alternatively, consists of an (iia) antibiotic having a reduced bacterial resistance combined or associated with an anti-inflammatory (iib) selected from among corticosteroids. The composition (i) being for use in a method for the pre-surgical or post-surgical treatment of bacterial infections in a patient to be subjected to eye surgery. Eye surgery may be for removing cataract. The composition (i) is administered in the eye of said patient, before and/or after surgery. Should the composition (i) be administered after surgery, the method of treatment provides for that said composition be administered via non-intravitreal route in the eye of said patient.

The antibiotic (iia) having reduced bacterial resistance is selected from among antibiotics belonging to the quinolone family of antibiotics, in particular fluoroquinolones such as levofloxacin.

The anti-inflammatory (iib) is selected from among compounds belonging to the corticosteroid family. Said family comprises compounds belonging to the dexamethasone group of compounds.

The Applicant found it useful to develop a composition in the form of eye drops which associates two active components, in the field of treatments for use in the prevention, reduction or elimination of pre-surgical and/or post-surgical bacterial infections. In the mixture contained in the composition, one of the two active components is an antibiotic, such as levofloxacin, which has had a lower environmental dispersion in the past years (reduced induction of bacterial resistance).

Table 1 below shows, as bacterial susceptibility, the changes recorded in the last twenty years in bacterial resistance to the main antibiotics (the greater the value, the lesser resistance there is for the antibiotic; Trade journal entitled "L'Oculista italiano", in the Special edition on Netilmicin, Year XLVIII - September 2016).

**Table 1**

| **Tab. 1** | **Resistance changes over time (Antibiotic susceptibility (%)]** | | | | |
|---|---|---|---|---|---|
| | **1995-2004** | | **2005-2014** | | |
| | **Adults** | **Children** | **Adults** | **Children** | |
| **Ampicillin** | **51.08** | **65.08** | **50.7** | **60.94** | |
| **Chloramphenicol** | **73.95** | **85.97** | **76.43** | **88.23** | |
| **Tetracycline** | **60.51** | **66.56** | **59.03** | **64.9** | |
| **Neomycin** | **35.71** | **20.78** | **38.27** | **21.72** | |
| **Netilmicin** | **63.6** | **48.52** | **63.55** | **50.88** | |
| **Amikacin** | **49.33** | **28.37** | **48.26** | **32.37** | |
| **Ciprofloxacin** | **83.44** | **84.35** | **71.49** | **84.59** | |
| **Moxifloxacin*** | **xxxx** | **xxxx** | **87.76** | **94.1** | |
| **Ofloxacin** | **75.42** | **74.79** | **68.68** | **79.93** | |
| **Gentamicin** | **51.4** | **34.49** | **41.41** | **29.69** | |
| **Tobramycin** | **45.51** | **24.37** | **34.84** | **22.79** | |
| **Norfloxacin** | **67.56** | **58.44** | **48.17** | **55.16** | |
| **Lomefloxacin** | **68.74** | **57.42** | **52.03** | **55.86** | |
| **Levofloxacin** | **xxx** | **xxx** | **83.45** | **93.23** | |

| | | | | | |
|---|---|---|---|---|---|
| *Susceptibility to antibiotics: 20 years of experience* | | | | | |

According to the data published in Table 1, levofloxacin should be considered one of the antibiotics with lower bacterial resistance. The levofloxacin molecule shows one of the lowest levels of antibiotic resistance.

Levofloxacin (CAS No: 100986-85-4) is a third-generation synthetic fluoroquinolone antibacterial agent that inhibits the supercoiling activity of the DNA bacterial gyrase, blocking DNA replication. This is the isomer (L) of ofloxacin. Levofloxacin appears as a light white-yellowish-yellow-white crystal or crystalline powder. It is poorly soluble in water and has a molecular weight of 361.4 g/mol.

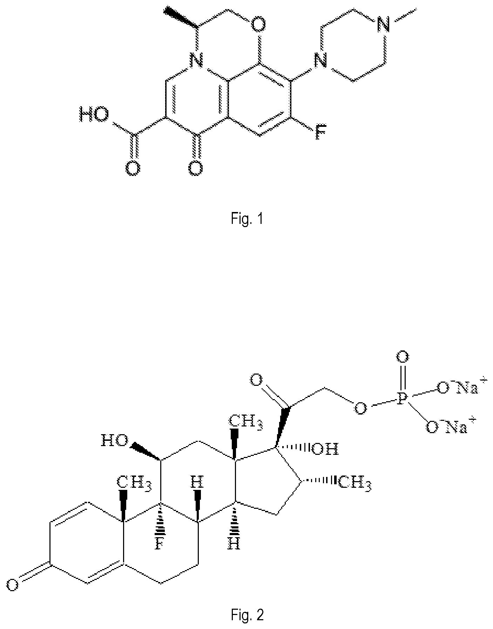

Levofloxacin, whose formula is shown in Figure 1, is more soluble in water than ofloxacin, norfloxacin or ciprofloxacin at neutral pH (Raizman et al., 2002; Koch et al., 2005). The solubility in water was 1.85% after 13 weeks of mixing (Robertson et al., 2005). It is sufficiently lipophilic to penetrate into the eyes (Keating, 2009).

Dexamethasone is a molecule belonging to the steroidal anti-inflammatories category, which may exist in insoluble or soluble lipophilic form, such as phosphate ester, a pro-drug of dexamethasone. In the present composition, for example in eye drops, dexamethasone is used as the phosphate ester, such as sodium phosphate ester or disodium phosphate ester [2-[(8S,9R,10S,11S,13S,14S,16R,17R)-9-fluoro-11,17-di-hydroxy-10,13,16-trimethyl-3-oxo-6,7,8,11,12,14,15,16-octahydrocyclopenta[a]phenanthren-17-yl]-2-oxoethyl]phosphate. The structural formula of dexamethasone sodium phosphate is shown in Figure 2.

Table 2 exemplifies the dosage per product in its common clinical practice with number of drops/day for each day of treatment for the days of treatment.

**Table 2**

| N° of drops | | | | | | | | | | | | | | |
|---|---|---|---|---|---|---|---|---|---|---|---|---|---|---|
| P | D 1 | D 2 | D 3 | D 4 | D 5 | D 6 | D 7 | D 8 | D 9 | D 10 | D 11 | D 12 | D 13 | D 14 |
| P1 | 4 | 4 | 4 | 4 | 4 | 4 | 4 | 4 | 4 | 4 | 4 | 4 | 4 | 4 |
| P2 | 4 | 4 | 4 | 4 | 4 | 4 | 4 | Not expected | | | | | | |
| P3 | 4 | 4 | 4 | 4 | 4 | 4 | 4 | 4 | 4 | 4 | 4 | 4 | 4 | 4 |

| | | | | | | | | | | | | | | |
|---|---|---|---|---|---|---|---|---|---|---|---|---|---|---|
| P= Product; D= Day. P1= Maxidex (Desametasone) having composition published as follows: Dexamethasone 0.1% (1 mg/ml). Benzalkonium chloride 0.01% (0.2 mg/ml). Hydroxypropyl methylcellulose, water. Dosage: 1 drop every 4 hours. In case of severe inflammations 1 or 2 drops every 30 or 60 minutes until resolution of symptoms. Dosage should be reduced gradually. P2= Composition Example 1 -Levo/dexa. P3= Tobradex^{®} having composition published as follows: Tobramycin 0.3% (3 mg/ml). Dexamethasone 0.1% (1 mg/ml). Benzalkonium chloride 0.01% (0.2 mg/ml). Excipients: tyloxapol, EDTA, sodium chloride, hydroxyethyl cellulose, sodium sulphate, sulfuric acid and/or sodium hydroxide, water. Dosage: 1 or 2 drops 4-5 times a day for the time prescribed by the physician. | | | | | | | | | | | | | | |

For the first time, use of levofloxacin is recommended for the pre- and post-eye surgical prevention of bacterial infections in patients subjected to surgery, such as cataract surgery. In addition, the dosage of Levofloxacin is reduced from 8 drops/day in the first two days to 4 drops/day in the first two days, as in the remaining five days of treatment. Compared to the treatment with P3 (Tobradex^{®}) of 14 days, P2 (Composition Example 1 -Levodexa) allows to avoid prolonged use of the antibiotic for more than 7 days.

Besides levofloxacin and dexamethasone, the composition of the present invention also comprises a preservative selected from among the common classes or in use, in particular quaternary ammonium salts whose preferred and most widely used is benzalkonium chloride and a buffer system such as phosphate buffers, citrate, borate or combination thereof, capable of stabilising the pH value of the composition at a value around neutral pH 7, comprised from 6 to 8, for example 6.2; 6.4; 6.6; 6.8; 7; 7.2; 7.4; 7.6; 7.8; 8.

Dexamethasone in its phosphate ester form is a molecule soluble at certain pH values. On the other hand, levofloxacin (shown in Figure 1) is in zwitterion form, that is it is an inner salt containing an anionic part and a cationic part, with a pKa1: 5.6; pKa2: 7.9, which means that the anion, cation, zwitterion forms of the molecule coexist in solution form. The balance between the different forms of Levofloxacin changes depending on the pH of the solution, which should preferably be maintained between 7 and 8 in order to stabilise the solubilisation of both the actives.

An embodiment refers to a treatment and a method of treatment which provides for the administration of an ophthalmic composition comprising a mixture comprising, a or alternatively, consisting of levofloxacin or a salt thereof or a hydrate or hemihydrate thereof such as levofloxacin hemihydrate, and dexamethasone or a salt thereof such as dexamethasone sodium phosphate or disodium phosphate. The concentration of levofloxacin, for example levofloxacin hemihydrate, is comprised from 1 mg/ml to 10 mg/ml of liquid composition, preferably it is comprised from 3 mg/ml to 7 mg/ml of liquid composition, even more preferably it is comprised from 4 mg/ml to 6 mg/ml of liquid composition, for example 4.5 mg/ml, or 5 mg/ml, or 5.5 mg/ml.

The concentration of dexamethasone or a salt thereof or a hemihydrate thereof or a hydrate thereof, for example dexamethasone sodium phosphate (Figure 2), is comprised from 0,25 mg/ml to 2,5 mg/ml of liquid composition, preferably it is comprised from 0,5 mg/ml to 2 mg/ml of liquid composition, even more preferably it is comprised from 1 mg/ml to 1,5 mg/ml of liquid composition, for example 1,2 mg/ml, or 1,3 mg/ml, or 1,4 mg/ml.

Further, the ophthalmic composition comprises one or more substances selected from among the group comprising or, alternatively, consisting of sodium phosphate, sodium phosphate monobasic monohydrate, sodium phosphate dibasic, sodium phosphate dibasic dodecahydrate, sodium citrate, sodium chloride, boric acid and/or borates, benzalkonium chloride, sodium hyaluronate or hyaluronic acid, NaOH 1 N for correcting the pH to a value comprised from 6,5 to 7,5, preferably 7, advantageously 7,2 and distilled water.

A preferred embodiment regards a treatment and a method of treatment which provides for the administration of the composition as reported in Example 1 shown in Table 3.

Forming an object of the present invention is a method of administration of said composition for use with the following dosage regimen:
- administration of said composition for use via non-intravitreal route to the eye of said patient on daily basis for a period of 7 days following the eye surgery, for a number of times or daily administrations comprised from 1 to 4 times or administrations/day, advantageously 3 or 4 times a day, preferably 4 times or administrations/day, of 1 or 2 drops (preferably 1 drop);
- preferably subsequent administration, for a period comprised from 3 to 7 days, of only one anti-inflammatory drug dexamethasone alone which allows to treat inflammatory events in a patient subjected to eye surgery, even more preferably without a subsequent administration of an antibiotic levofloxacin alone.

Reference to methods of treatment in the present disclosure are to be intended to refer to a composition for use in a method for preventing or treating post-surgery infections in a patient previously subjected to eye surgery.

Preferred embodiments E(n) of the present invention are listed herein after.

Preferably, the ophthalmic composition is in solution form and comprises a mixture comprising, or alternatively, consisting of an antibiotic, a levofloxacin or a salt thereof or a hydrate thereof or a hemihydrate thereof and a corticosteroid, a dexamethasone or a salt thereof or a hydrate thereof or a hemihydrate thereof, and optionally one or more pharmaceutical grade additives and excipients and water; said composition being for use in a method for treating post-surgery infections in a patient previously subjected to eye surgery; wherein said composition is administered via non-intravitreal route to the eye of said patient on daily basis for a period of 7 days following the eye surgery.

Preferably, said eye surgery is a cataract surgery.

Preferably, said method for the treatment of post-surgery infections in a patient previously subjected to eye surgery provides for the administration of said composition via the ocular topical route.

Preferably, said levofloxacin or a salt thereof or a hemihydrate thereof or a hydrate thereof is present in said composition in solution form at a concentration comprised from 1 mg/ml to 10 mg/ml of liquid composition, preferably it is comprised from 3 mg/ml to 7 mg/ml of liquid composition, even more preferably it is comprised from 4 mg/ml to 6 mg/ml of liquid composition, for example 4,5 mg/ml, or 5 mg/ml, or 5,5 mg/ml.

Preferebly, said dexamethasone or a salt thereof or a hemihydrate thereof or a hydrate thereof is present in said composition in solution form at a concentration comprised from 0,25 mg/ml to 2.5 mg/ml of liquid composition, preferably it is comprised from 0,5 mg/ml to 2 mg/ml of liquid composition, even more preferably it is comprised from 1 mg/ml to 1,5 mg/ml of liquid composition, for example 1,2 mg/ml, or 1,3 mg/ml, or 1,4 mg/ml.

Preferably, said ophthalmic composition further comprises one or more pharmaceutical grade additives and excipients selected from among the group comprising or, alternatively, consisting of sodium phosphate, sodium phosphate monobasic monohydrate, sodium phosphate dibasic, sodium phosphate dibasic dodecahydrate, sodium citrate, sodium chloride, boric acid and/or borates, benzalkonium chloride, sodium hyaluronate or hyaluronic acid, NaOH 1 N for correcting the pH to a value comprised from 6,5 to 7,5, preferably 7, or 7,2 and distilled water.

Preferably, said composition is administered in the eye of said patient on daily basis for a period of 7 days following the eye surgery for a number of daily administrations comprised from 1 to 4 administrations/day, 1 or 2 drops of said composition, preferably 4 administrations/day, of 1 drop of said composition.

Preferably, said method for the preventive or curative treatment of post-surgical infections in a patient previously subjected to eye surgery does not provide for a subsequent administration of an antibiotic levofloxacin alone, but provides for a subsequent administration of an anti-inflammatory dexamethasone alone.

### EXAMPLE 1

**Table 3**

| Components | Quantity (mg/ml) | Function |
|---|---|---|
| Levofloxacin hemihydrate | 5.12 (equivalent to 5 of levofloxacin) | Active |
| Dexamethasone sodium phosphate Sodium phosphate monobasic monohydrate | 1.32 (equivalent to 1 dexamethasone) | Active |
| Dibasic sodium phosphate dodecahydrate | | |
| Sodium citrate | 1.47 | Buffer agent |
| Benzalkonium chloride 50 % solution | | |
| NaOH 1N | 10.0 | Buffer agent |
| Distilled water | 21.0 | Buffer agent |
| | 0.10 | Preservative |
| | q.s. at pH 7.2 | PH modifier |
| | q.s. at 1 ml | Diluent |

The composition of Example 1 in Table 3 is a transparent greenish solution at pH 7.2 and it has the main chemical physical specifications reported in Table 4.

**Table 4**

| Appearance | Clear solution |
|---|---|
| Colour of the solution | ≤ GY3 |
| pH | 7.2 (± 0.2) |
| Osmolarity | 300 mOsm/Kg (± 30) |

Forming an object of the present invention is a process for preparing the composition (i), preferably in a pharmaceutical form as eye drops, by means of methods, apparatus and instruments known to the man skilled in the art of sterile ophthalmic solutions.

The ophthalmic composition (i) subject of the present invention is a sterile composition.

The process for preparing the composition (i) of the present invention comprises adding and dissolving the components of Example 1 in water for injection in a reduced and controlled contamination area.

The aforementioned dissolution step is followed by an aseptic filtration step using a sterilising filter having a porosity of about 0.2 µm capable of retaining all microorganisms.

The aforementioned filtration step is followed by a step of filling/sealing the composition in the form of a sterile solution in pre-sterilised ophthalmic containers; said containers being provided with a dropper device and a cap closure device. The filling/sealing step is carried out under almost no contamination conditions, so-called class A area.

The composition in the form of a solution is filtered under sterile conditions and packaged in a plastic (primary) container made of opaque polyethylene, since levofloxacin is photosensitive. Said container is provided with a dropper which dispenses 30 microliters (0.03 ml) per drop.

The primary container of the eye drops solution is a light-opaque plastic bottle, conveniently made of low-density polyethylene (LDPE) containing 50 µlitres of solution, provided with an opaque LDPE dropper and a high-density polyethylene (HDPE) screw cap.

Other embodiments of the composition of the present invention are described in Examples 2 (Table 5), 3 (Table 6) and 4 (Table 7) below.

### EXAMPLE 2 MULTI-DOSE FORMULA WITHOUT PRESERVATIVE

5 ml (from 3 to 10 ml) of solution having the following composition.

**Table 5**

| Components | Quantity (mg/ml) | Function |
|---|---|---|
| Levofloxacin hemihydrate | 5.12 (equivalent to 5 of levofloxacin) | Active |
| Dexamethasone sodium phosphate | 1.32 (equivalent to 1 dexamethasone) | Active |
| Sodium phosphate monobasic monohydrate | 1.47 | Buffer agent |
| Dibasic sodium phosphate dodecahydrate | 10.0 | Buffer agent |
| Sodium citrate | 21.0 | Buffer agent |
| NaOH 1N | q.s. at pH 7.2 | PH modifier |
| Distilled water | q.s. at 1 ml | Diluent |

### EXAMPLE 3 SINGLE-DOSE FORMULA WITHOUT PRESERVATIVE

A volume of 0. 4 ml (from 0. 1 to 1 ml) of solution having the following composition.

**Table 6**

| Components | Quantity (mg/ml) | Function |
|---|---|---|
| Levofloxacin hemihydrate | 5.12 (equivalent to 5 of levofloxacin) | Active |
| Dexamethasone sodium phosphate | 1.32 (equivalent to 1 dexamethasone) | Active |
| Sodium phosphate monobasic monohydrate | 1.47 | Buffer agent |
| Dibasic sodium phosphate | 10.0 | Buffer agent |
| dodecahydrate | | |
| Sodium citrate | 21.0 | Buffer agent |
| NaOH 1N | q.s. at pH 7.2 | PH modifier |
| Distilled water | q.s. at 1 ml | Diluent |

### EXAMPLE 4 VISCOSIFIED FORMULA FOR BETTER BIOADHESION

**Table 7**

| Components | Quantity (mg/ml) | Function |
|---|---|---|
| Levofloxacin hemihydrate | 5.12 (equivalent to 5 of levofloxacin) | Active |
| Dexamethasone sodium phosphate | 1.32 (equivalent to 1 dexamethasone) | Active |
| Sodium phosphate monobasic monohydrate | 1.47 | Buffer agent |
| Dibasic sodium phosphate dodecahydrate | 10.0 | Buffer agent |
| Sodium citrate | 21.0 | Buffer agent |
| NaOH 1N | q.s. at pH 7.2 | PH modifier |
| Sodium hyaluronate | 0.3% | viscosifier |
| Distilled water | q.s. at 1 ml | Diluent |

The solutions of Examples 1-4 of the present invention have all been tested. For the sake of simplicity, herein reported is only one clinical study conducted *in vivo* in humans with the solution of Example 1. Patients were selected to reflect a representative case study of subjects subjected to cataract surgery. In the clinical study, the solution of Example 1 was compared with the solution of Tobradex^{®} eye drops in post-cataract surgery treatment.

The treatment commonly used and dedicated to patients subjected to cataract surgery provides for 15 days of therapy for the prevention of infections associated with cataract surgery in the adult (15 days post-surgical treatment).

A clinical study was carried out with the following premises:
(a) The indications are phlogistic states (post-surgical and non-surgical) in the presence or at risk of infection.
(b) The clinical efficacy assessment is mainly (almost exclusively) guided by the anti-inflammatory activity at the anterior chamber level.
(c) Thus, in a comparative clinical trial between a levofloxacin/dexamethasone association or combination and a reference drug (Tobradex^{®}) in post-operative phlogosis, the main endpoint can only be of the inflammatory type; considering the same steroid present in the two compared drugs (dexamethasone), only one non-inferiority study can be hypothesized.

The international multicentre *in-vivo* study conducted on 808 patients with 2 treatment arms provides for a first treatment arm with Tobradex^{®} and a second treatment arm with the composition of Example 1, subject of the present invention. The primary objective of the study was to demonstrate the non-inferiority of a levofloxacin and dexamethasone association/combination treatment administered for a period of 7 consecutive days followed, said treatment, by another dexamethasone-only treatment for another 7 consecutive days with respect to 14 consecutive days of a single treatment administered with the Tobradex^{®} eye drops alone, combination of tobramycin and dexamethasone. The study protocol then followed the treatment scheme shown in Figure 3.

Levofloxacin-dexamethasone arm dosage: levofloxacin-dexamethasone 1 drop 4 times a day for 7 days followed by 1 drop of Maxidex (dexamethasone) 4 times a day.

Tobradex arm dosage: 1 drop 4 times a day for 15 days.

Maxidex (Dexamethasone) having composition published as follows:
Dexamethasone 0.1% (1 mg/ml).
Benzalkonium chloride 0.01% (0.2 mg/ml).
Hydroxypropyll methylcellulose, water.
Dosage:
   1 drop every 4 hours. In case of severe inflammations 1 or 2 drops every 30 or 60 minutes until resolution of symptoms. Dosage should be reduced gradually.

Levofloxacin-dexamethasone composition as in example 2.

Tobradex^{®} having composition published as follows
Tobramycin 0.3% (3 mg/ml).
Dexamethasone 0.1% (1 mg/ml).
Benzalkonium chloride 0.01% (0.2 mg/ml).
Excipients: tyloxapol (Tyloxapol is a non-ionic liquid polymer of the aryl alkyl polyether alcohol alcohol-type and it is used as a surfactant), EDTA, sodium chloride, hydroxyethyl cellulose, sodium sulphate, sulfuric acid and/or sodium hydroxide, water.

The study was conducted and completed successfully. The study demonstrated the non-inferiority of a treatment administered with the levofloxacin and dexamethasone association/combination (according to the present invention) with respect to a treatment administered with the tobramycin and dexamethasone association/combination (Tobradex^{®}), both of said treatments having been administered to patients after cataract surgery.

The study confirmed that the dose of the antibiotic levofloxacin administered to each patient for 7 days is markedly lower than the dose of tobramycin administered for 14 days.

The treatment of the present invention reduces the need for antibiotic doses with a lower number of total drops administered on the intended treatment days, generating less environmental dispersion of the antibiotic and, therefore, less resistance/sensitizations to levofloxacin.

Furthermore, there was observed a more prompt remission of the inflammation observable on the symptoms as early as the third day with respect to the treatment administered with the tobramycin and dexamethasone association/combination (Tobradex^{®}), considering the same number of administered drops.

Advantageously, the treatment administered with the levofloxacin and dexamethasone association/combination entails a reduction of antibiotic treatment days from 14 to 7 while maintaining the same results obtained with a treatment administered, considering the same conditions, with the tobramycin and dexamethasone association/combination (Tobradex^{®}) in terms of inflammatory remission (Primary endpoints). Furthermore, it was however observed that with both products, considerable results were obtained already after the first 3-4 days as regards the remission of the related inflammatory events. Surprisingly, there is no need for extending the therapy at the end of treatment with the composition of the present invention with other 7 days of administration of the corticosteroid dexamethasone alone.

The results are reported in Table 8 below.

The table below summarises the comparative results in % of the study endpoints for Levofloxacin-dexamethasone (LD) and Tobramycin-dexamethasone (TD) only.

**Table 8**

| % of patients | SCREENING | | D 4 | | D 8 | | D 15 | |
|---|---|---|---|---|---|---|---|---|
| | LD | TD | LD | TD | LD | TD | LD | TD |
| No cells under the Slit lamp | 100 | 100 | 73.1 | 76.8 | 85.7 | 86.7 | 95.1 | 94.9 |
| Aqueous flare | 100 | 100 | 86.2 | 86.9 | 96.2 | 94.9 | 99.2 | 98.9 |
| Hyperaemia | 100 | 100 | 85.3 | 82.1 | 88.1 | 91.1 | 93.9 | 95.4 |
| Total ocular symptom score | 100 | 100 | 78.4 | 75.1 | 81.0 | 81.6 | 86.8 | 89.3 |

| | | | | | | | | |
|---|---|---|---|---|---|---|---|---|
| D=Day | | | | | | | | |

### PRIMARY ENDPOINTS

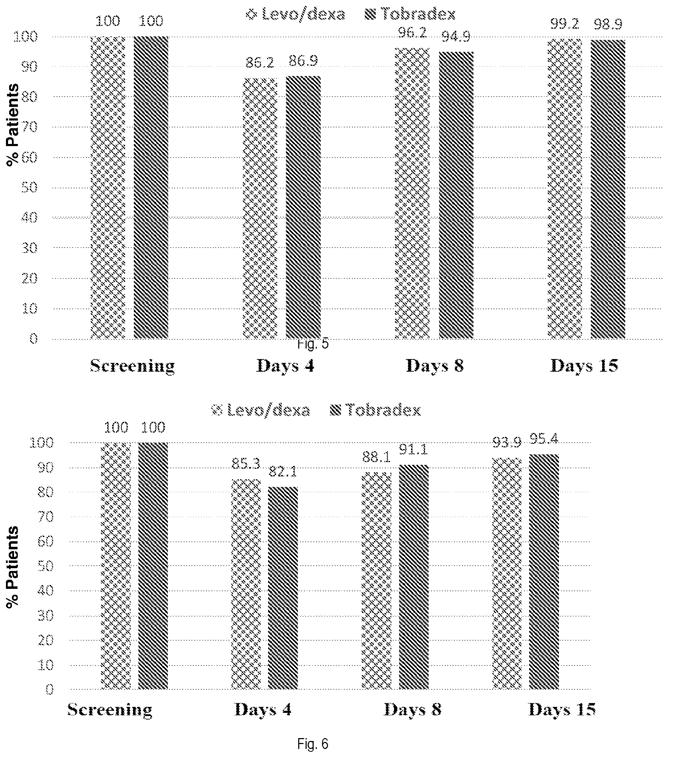

In case of inflammations, inflammatory cells and material ("Flare") can be observed in the eye. The examination under the *"slit lamp"* allows to count and quantify inflammatory events.
Signs: detection of cells under the *slit lamp.* No cells under the *slit lamp* (Figure 4)
Signs: detection of material under the *slit lamp* (Figure 5)
AQUEOUS FLARE: proteins present in the aqueous humour following the inflammatory process.

### SECONDARY ENDPOINTS

Signs: Percentage of patients with hyperaemia at different stages of the study (Figure 6).

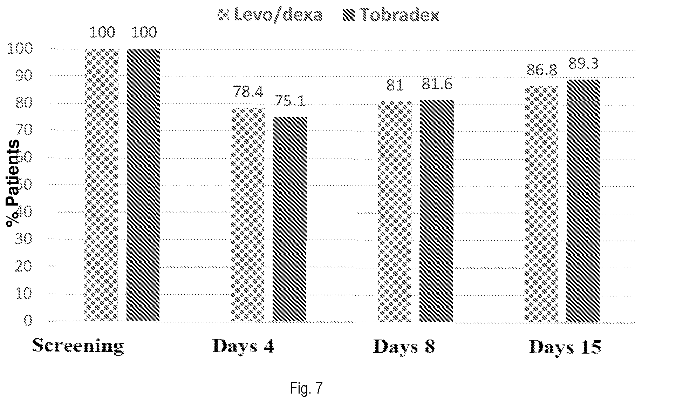

Symptoms: Percentage of patients suffering from discomfort/irritation at different stages of the study (Figure 7).

## Claims

1. An ophthalmic composition in solution form comprising a mixture comprising, or alternatively, consisting of an antibiotic, said antibiotic being levofloxacin or a salt thereof or a hydrate thereof or a hemihydrate thereof and a corticosteroid, said corticosteroid being dexamethasone or a salt thereof or a hydrate thereof or a hemihydrate thereof, and optionally one or more pharmaceutical grade additives and excipients and water; said composition being for use in a method for preventing or treating post-surgery infections in a patient previously subjected to eye surgery;
wherein said composition is administered via non-intravitreal route to the eye of said patient on daily basis for a period of 7 days following the eye surgery.

2. The composition for use according to claim 1, wherein said eye surgery is a cataract surgery.

3. The composition for use according to claims 1 or 2, wherein said method for the treatment of post-surgery infections in a patient previously subjected to eye surgery provides for the administration of said composition via the ocular topical route.

4. The composition for use according to any one of the preceding claims, wherein said levofloxacin or a salt thereof or a hemihydrate thereof or a hydrate thereof is present in said composition in solution form at a concentration comprised from 1 mg/ml to 10 mg/ml of liquid composition, preferably it is comprised from 3 mg/ml to 7 mg/ml of liquid composition, even more preferably it is comprised from 4 mg/ml to 6 mg/ml of liquid composition, for example 4,5 mg/ml, or 5 mg/ml, or 5,5 mg/ml.

5. The composition for use according to any one of the preceding claims, wherein said dexamethasone or a salt thereof or a hemihydrate thereof or a hydrate thereof is present in said composition in solution form at a concentration comprised from 0,25 mg/ml to 2.5 mg/ml of liquid composition, preferably it is comprised from 0,5 mg/ml to 2 mg/ml of liquid composition, even more preferably it is comprised from 1 mg/ml to 1,5 mg/ml of liquid composition, for example 1,2 mg/ml, or 1,3 mg/ml, or 1,4 mg/ml.

6. The composition for use according to any one of the preceding claims, wherein said ophthalmic composition further comprises one or more pharmaceutical grade additives and excipients selected from among the group comprising or, alternatively, consisting of sodium phosphate, sodium phosphate monobasic monohydrate, sodium phosphate dibasic, sodium phosphate dibasic dodecahydrate, sodium citrate, sodium chloride, boric acid and/or borates, benzalkonium chloride, sodium hyaluronate or hyaluronic acid, NaOH 1 N for correcting the pH to a value comprised from 6,5 to 7,5, preferably 7, or 7,2 and distilled water.

7. The composition for use according to any one of the preceding claims, wherein said composition is administered in the eye of said patient on daily basis for a period of 7 days following the eye surgery for a number of daily administrations comprised from 1 to 4 administrations/day, 1 or 2 drops of said composition, preferably 4 administrations/day, of 1 drop of said composition.

8. The composition for use according to any one of the preceding claims, wherein said method for the preventive or curative treatment of post-surgical infections in a patient previously subjected to eye surgery does not provide for a subsequent administration of an antibiotic levofloxacin alone.

## Patentansprüche

1. Ophthalmische Zusammensetzung in gelöster Form, umfassend eine Mischung, umfassend oder alternativ bestehend aus einem Antibiotikum, wobei das Antibiotikum Levofloxacin oder ein Salz davon oder ein Hydrat davon oder ein Hemihydrat davon und ein Corticosteroid ist, wobei das Corticosteroid Dexamethason oder ein Salz davon oder ein Hydrat davon oder ein Hemihydrat davon ist, und gegebenenfalls einen(m) oder mehrere(n) pharmazeutische(n) Zusatzstoffe(n) und Hilfsstoffe(n) und Wasser; wobei die Zusammensetzung zur Verwendung in einem Verfahren zur Vorbeugung oder Behandlung von postoperativen Infektionen bei einem Patienten bestimmt ist, der zuvor einer Augenoperation unterzogen wurde;
wobei die Zusammensetzung über einen Zeitraum von 7 Tagen nach der Augenoperation täglich über einen nicht intravitrealen Weg an das Auge des Patienten verabreicht wird.

2. Zusammensetzung zur Verwendung nach Anspruch 1, wobei die Augenoperation eine Kataraktoperation ist.

3. Zusammensetzung zur Verwendung nach Anspruch 1 oder 2, wobei das Verfahren zur Behandlung von postoperativen Infektionen bei einem Patienten, der zuvor einer Augenoperation unterzogen wurde, die Verabreichung der Zusammensetzung über den topischen Weg des Auges vorsieht.

4. Zusammensetzung zur Verwendung nach einem der vorhergehenden Ansprüche, wobei das Levofloxacin oder ein Salz davon oder ein Hemihydrat davon oder ein Hydrat davon in der Zusammensetzung in gelöster Form in einer Konzentration von 1 mg/ml bis 10 mg/ml flüssiger Zusammensetzung vorliegt, vorzugsweise von 3 mg/ml bis 7 mg/ml flüssiger Zusammensetzung umfasst ist, noch bevorzugter von 4 mg/ml bis 6 mg/ml flüssiger Zusammensetzung umfasst ist, zum Beispiel 4,5 mg/ml oder 5 mg/ml oder 5,5 mg/ml.

5. Zusammensetzung zur Verwendung nach einem der vorhergehenden Ansprüche, wobei das Dexamethason oder ein Salz davon oder ein Hemihydrat davon oder ein Hydrat davon in der Zusammensetzung in gelöster Form in einer Konzentration von 0,25 mg/ml bis 2,5 mg/ml flüssiger Zusammensetzung vorliegt, vorzugsweise von 0,5 mg/ml bis 2 mg/ml flüssiger Zusammensetzung umfasst ist, noch bevorzugter von 1 mg/ml bis 1,5 mg/ml flüssiger Zusammensetzung umfasst ist, zum Beispiel 1,2 mg/ml oder 1,3 mg/ml oder 1,4 mg/ml.

6. Zusammensetzung zur Verwendung nach einem der vorhergehenden Ansprüche, wobei die ophthalmische Zusammensetzung ferner einen oder mehrere pharmazeutische Zusatzstoffe und Hilfsstoffe umfasst, die aus der Gruppe ausgewählt sind, die Natriumphosphat, einbasisches Natriumphosphat-Monohydrat, zweibasisches Natriumphosphat, zweibasisches Natriumphosphat-Dodecahydrat, Natriumcitrat, Natriumchlorid, Borsäure und/oder Borate, Benzalkoniumchlorid, Natriumhyaluronat oder Hyaluronsäure, NaOH 1 N zum Korrigieren des pH-Werts auf einen Wert von 6,5 bis 7,5, vorzugsweise 7 oder 7,2 und destilliertes Wasser umfasst oder alternativ daraus besteht.

7. Zusammensetzung zur Verwendung nach einem der vorhergehenden Ansprüche, wobei die Zusammensetzung dem Auge des Patienten täglich für einen Zeitraum von 7 Tagen nach der Augenoperation für eine Anzahl von täglichen Verabreichungen von 1 bis 4 Verabreichungen/Tag, 1 oder 2 Tropfen der Zusammensetzung, vorzugsweise 4 Verabreichungen/Tag, von 1 Tropfen der Zusammensetzung verabreicht wird.

8. Zusammensetzung zur Verwendung nach einem der vorhergehenden Ansprüche, wobei das Verfahren zur vorbeugenden oder kurativen Behandlung von postoperativen Infektionen bei einem Patienten, der zuvor einer Augenoperation unterzogen wurde, keine nachfolgende Verabreichung des Antibiotikums Levofloxacin allein vorsieht.

## Revendications

1. Composition ophtalmique sous forme de solution comprenant un mélange comprenant, ou en variante, constitué d'un antibiotique, ledit antibiotique étant la lévofloxacine ou un sel de celle-ci, ou un hydrate de celle-ci, ou un hémihydrate de celle-ci , et un corticostéroïde, ledit corticostéroïde étant la dexaméthasone ou un sel de celle-ci, ou un hydrate de celle-ci, ou un hémihydrate de celle-ci, et, éventuellement, un ou plusieurs additifs et excipients de qualité pharmaceutique et de l'eau ; ladite composition étant destinée à être utilisée dans un procédé de prévention ou de traitement des infections postopératoires chez un patient ayant préalablement subi une chirurgie oculaire ;
dans laquelle ladite composition est administrée par voie non intravitréenne à l'œil dudit patient quotidiennement pendant une période de 7 jours suivant la chirurgie oculaire.

2. Composition destinée à être utilisée selon la revendication 1, dans laquelle ladite chirurgie oculaire est une chirurgie de la cataracte.

3. Composition destinée à être utilisée selon les revendications 1 ou 2, dans laquelle ledit procédé de traitement des infections postopératoires chez un patient ayant préalablement subi une chirurgie oculaire prévoit l'administration de ladite composition par voie topique oculaire.

4. Composition destinée à être utilisée selon l'une quelconque des revendications précédentes, dans laquelle ladite lévofloxacine, ou un sel de celle-ci, ou un hémihydrate de celle-ci, ou un hydrate de celle-ci, est présente dans ladite composition sous forme de solution à une concentration comprise de 1 à 10 mg/ml de composition liquide, de préférence comprise de 3 à 7 mg/ml de composition liquide, et, plus préférablement encore, comprise de 4 à 6 mg/ml de composition liquide, par exemple 4,5 mg/ml, ou 5 mg/ml, ou 5,5 mg/ml.

5. Composition destinée à être utilisée selon l'une quelconque des revendications précédentes, dans laquelle ladite dexaméthasone, ou un sel de celle-ci, ou un hémihydrate de celle-ci, ou un hydrate de celle-ci, est présente dans ladite composition sous forme de solution à une concentration comprise entre 0,25 et 2,5 mg/ml de composition liquide, de préférence comprise entre 0,5 et 2 mg/ml de composition liquide, et, plus préférablement encore, comprise entre 1 et 1,5 mg/ml de composition liquide, par exemple 1,2 mg/ml, ou 1,3 mg/ml, ou 1,4 mg/ml.

6. Composition destinée à être utilisée selon l'une quelconque des revendications précédentes, dans laquelle ladite composition ophtalmique comprend en outre un ou plusieurs additifs et excipients de qualité pharmaceutique choisis parmi le groupe comprenant ou, en variante, constitué par le phosphate de sodium, le phosphate de sodium monobasique monohydraté, le phosphate de sodium dibasique, le phosphate de sodium dibasique dodécahydraté, le citrate de sodium, le chlorure de sodium, l'acide borique et/ou les borates, le chlorure de benzalkonium, l'hyaluronate de sodium ou l'acide hyaluronique, le NaOH 1 N pour corriger le pH à une valeur comprise de 6,5 à 7,5, de préférence 7 ou 7,2, et de l'eau distillée.

7. Composition destinée à être utilisée selon l'une quelconque des revendications précédentes, dans laquelle ladite composition est administrée dans l'œil dudit patient quotidiennement pendant une période de 7 jours suivant la chirurgie oculaire, à raison d'un nombre d'administrations quotidiennes compris de 1 à 4 administrations par jour, à raison de 1 ou 2 gouttes de ladite composition, de préférence 4 administrations par jour, d'1 goutte de ladite composition.

8. Composition destinée à être utilisée selon l'une quelconque des revendications précédentes, dans laquelle ledit procédé de traitement préventif ou curatif des infections postopératoires chez un patient ayant préalablement subi une chirurgie oculaire ne prévoit pas l'administration ultérieure de la lévofloxacine seule en tant qu'antibiotique.
